# EUROPEAN PATENT APPLICATION

(11) **EP 2 716 752 A1**
(43) Date of publication of application: **09.04.2014**
(21) Application number: 12790131.2
(22) Date of filing: 26.04.2012
(51) Int. Cl.: C12N 7/01, C07K 14/11, C12N 15/44, C12N 15/63, A61K 39/145, A61P 31/16, C12R 1/93

(54) **CANINE INFLUENZA RECOMBINANT VIRUS, PREPARATION METHOD THEREFOR AND APPLICATION THEREOF**

(30) Priority: 26.05.2011 CN 201110139528
(71) Applicant: Shanghai Veterinary Research Institute, CAAS, Shanghai 200241 (CN)
(72) Inventor: LI, Zejun, Shanghai 200241 (CN); TENG, Qiaoyang, Shanghai 200241 (CN)
(74) Representative: Sun, Yiming
(86) International application number: PCT/CN2012/074722
(87) International publication number: WO 2012/159522

(57) **Abstract**

Disclosed is a canine influenza recombinant virus. The recombinant virus comprises HA and NA genes of ZJCIV canine influenza virus as well as six internal genes PA, PB1, PB2, M, NP and NS of a PR8 virus. Also disclosed is a preparation method for the canine influenza recombinant virus and an application of the canine influenza recombinant virus. The canine influenza recombinant virus in the present invention can generate very high virus titer and hemagglutination titer on both a chicken embryo and an MDCK cell, and can be used as a good seed virus for developing canine influenza vaccines.

## Description

### FIELD OF THE INVENTION

The present invention relates to the technical field of bioengineering, in particular to a canine influenza recombinant virus and a preparation method therefore and an application thereof.

### BACKGROUND OF THE INVENTION

Influenza A virus is a major infectious disease that poses a threat to human health. Influenza viruses possess strict host specificity, so even spreading of the same virus on different hosts is still restricted by the host factor. Back in 2004, it was first reported in the United States that a major canine influenza outbreak was caused by an H3N8 subtype canine influenza virus, and it is found through sequence analysis that this subtype canine influenza virus derives from evolution of an equine influenza virus. Subsequently, equine influenza in Australia was also followed by a canine influenza outbreak. A canine influenza outbreak caused by an H3N2 subtype canine influenza virus began in Korea in 2008, and it is found through sequence analysis that this H3N2 subtype canine influenza virus is an avian-origin virus, which is different from the equine-origin canine influenza viruses in the United States and Europe.

Several H3N2 subtype canine influenza viruses were in vivo isolated from virus-infected canines in South China from 2006 to 2007, and it is found through sequence analysis that these viruses are highly homologous with the isolated virus in Korea. Surveys on pet dogs' serums in South China found that 6.7% of these dog serums are positive for influenza. In 2010, a canine influenza virus was also isolated from canines in East China by our lab and named as A/canine/Zhejiang/01/2010 (H3N2 subtype, ZJCIV for short). It is found through complete genome sequence analysis for the ZJCIV virus that this virus is highly homologous with the canine influenza viruses from South China and the H3N2 canine influenza virus from Korea. According to animal infection experiments, the ZJCIV virus is infectious for canines, and could cause a canine disease that is manifested as loss of appetite, hyperpyrexia, cough, nasal drainage of purulent secretions and other symptoms, and pulmonary congestion, pulmonary hemorrhage and inflammatory exudate-filled pulmonary alveoli are found after dissection.

A canine influenza virus inactivated vaccine was successfully developed by Intervet Company in June 2009 and has been commercially available in the United States, however, there is a difference in antigenicity between the pandemic canine influenza virus in China and the pandemic canine influenza virus in the United States, these two viruses originate from different branches of an H3 influenza virus. Thus, development of vaccines against H3N2 subtype canine influenza virus pandemic strains is of important and practical significance to prevention and control for canine influenza.

### SUMMARY OF THE INVENTION

The technical problem to be solved by the present invention is to provide a canine influenza recombinant virus. The canine influenza recombinant virus comprises: HA and NA genes of ZJCIV canine influenza virus as well as six internal genes of a PR8 virus, and the recombinant virus can be prepared into vaccines that are effectively against H3N2 subtype canine influenza virus.

In addition, a preparation method for the canine influenza recombinant virus and an application of the canine influenza recombinant virus also need to be provided.

To solve the technical problems above, the present invention is conducted as follows:

In one aspect, the present invention provides a canine influenza recombinant virus, which comprises HA and NA genes of ZJCIV canine influenza virus as well as six internal genes PA, PB1, PB2, M, NP and NS of a PR8 virus.

The nucleotide sequence of the HA gene of the canine influenza virus is selected from the group consisting of:
(1) a nucleotide sequence encoding an amino acid sequence of SEQ ID NO.1;
(2) a nucleotide sequence encoding an amino acid sequence which has at least 98% sequence identity to the amino acid sequence of SEQ ID NO.1;
the nucleotide sequence of the NA gene of the canine influenza virus is selected from the group consisting of:
(1) a nucleotide sequence encoding an amino acid sequence of SEQ ID NO.2;
(2) a nucleotide sequence encoding an amino acid sequence which has at least 98% sequence identity to the amino acid sequence of SEQ ID NO.2.

Preferably, the HA gene of the canine influenza virus has a nucleotide sequence of SEQ ID NO.3, or the HA gene of the canine influenza virus has a sequence having at least 98% sequence identity to the nucleotide sequence of SEQ ID NO.3.

Preferably, the NA gene of the canine influenza virus has a nucleotide sequence of SEQ ID NO.4, or the NA gene of the canine influenza virus has a sequence having at least 98% sequence identity to the nucleotide sequence of SEQ ID NO.4.

In the present invention, the amino acid sequence having more than 98% of homology with the amino acid sequence of SEQ ID NO.1 comprises an amino acid sequences having the hemagglutinin (HA) activity of the ZJCIV canine influenza virus, derived from deletion, addition, insertion or substitution of one or more amino acids in the amino acid sequence of SEQ ID NO.1.

In the present invention, the amino acid sequence having more than 98% of homology with the amino acid sequence of SEQ ID NO.2 comprises an amino acid sequences having the neuraminidase (NA) activity of the ZJCIV canine influenza virus, derived from deletion, addition, insertion or substitution of one or more amino acids in the amino acid sequence of SEQ ID NO.2.

HA and NA are two important surface antigens of an influenza virus, and antigenic variation of this influenza virus refers mainly to variation of HA and NA, in particular, faster variation occurs in HA. Therefore, the HA gene of the ZJCIV canine influenza virus in the canine influenza recombinant virus of the present invention is a nucleotide sequence encoding the amino acid sequence of SEQ ID NO.1, or a nucleotide sequence encoding an amino acid sequence having more than 98% of homology with the amino acid sequence of SEQ ID NO.1; the NA gene of the ZJCIV canine influenza virus in the canine influenza recombinant virus of the present invention is a nucleotide sequence encoding the amino acid sequence of SEQ ID NO.2, or a nucleotide sequence encoding an amino acid sequence having more than 98% of homology with the amino acid sequence of SEQ ID NO.2.

In another aspect, the present invention further provides a preparation method for the canine influenza recombinant virus, the method comprising:
constructing recombinant plasmids comprising the HA and NA genes of the ZJCIV canine influenza virus respectively;
transfecting the recombinant plasmids of the HA and NA genes, and six plasmids comprising the internal genes PA, PB1, PB2, M, NP and NS of the PR8 virus respectively, to a 293T cell, and culturing the transfected cell;
inoculating the cultured cell supernatant to a chicken embryo, culturing the chicken embryo in an incubator for a proper time period to obtain chicken embryo allantoic fluid, detecting the hemagglutination condition of the allantoic fluid, and at the presence of hemagglutinin activity, determining the absence of unexpected variations by sequencing to obtain the canine influenza recombinant virus.

Preferably, the cultured cell supernatant is inoculated to a 9-day to 11-day chicken embryo, and the chicken embryo is cultured in a 37°C incubator for 48-72 hours to obtain chicken embryo allantoic fluid.

PBD vectors are used as empty vectors in the recombinant plasmids.

Still in another aspect, the present invention further provides an application of the canine influenza recombinant virus for preventing or treating canine influenza.

Still in another aspect, the present invention further provides an influenza vaccine comprising the canine influenza recombinant virus.

Preferably, the influenza vaccine further comprises adjuvant.

Still in another aspect, the present invention further provides a method of preventing or treating canine influenza , the method comprising administering the influenza vaccine to a subject.

The canine influenza recombinant virus in the present invention can generate very high virus titer and hemagglutination titer on both a chicken embryo and an MDCK cell, and can be used as a good seed virus for developing canine influenza vaccines.

### BRIEF DESCRIPTION OF THE DRAWINGS

The present invention will be further described below in details with reference to the accompanying drawings and the embodiments.
Fig.1 is an RT-PCR electrophoretogram of the HA and NA of the ZJCIV canine influenza virus in the embodiment 1 of the present invention;
Fig.2 is a graph illustrating hemagglutination titers at different time after inoculation of the rescued recombinant virus and the ZJCIV to a chicken embryo in the embodiment 3 of the present invention;
Fig.3 is a graph illustrating growth curve comparison of the rescued recombinant virus and the ZJCIV on the chicken embryo in the embodiment 3 of the present invention;
Fig.4 is a graph illustrating hemagglutination titers at different time after inoculation of the rescued recombinant virus and the ZJCIV to an MDCK cell in the embodiment 3 of the present invention;
Fig.5 is a graph illustrating growth curve comparison of the rescued recombinant virus and the ZJCIV on the MDCK cell in the embodiment 3 of the present invention.

### DETAILED DESCRIPTION OF THE INVENTION

In the embodiments described hereinafter, experimental methods in which specific conditions are unspecified are typically carried out under general conditions, e.g. the method described in Short Protocols in Human Genetics (Edited by Ausubel F. M., Kingston R. E., Seidman J. G. et al., Translated by MA, Xuejun, SHU, Yuelong. Beijing: Science Press, 2004).

One of the critical prerequisites for developing canine influenza virus vaccines is good seed virus, and the A/canine/Zhejiang/01/2010 virus (H3N2 subtype, ZJCIV for short) that is previously isolated in the lab has extremely low hemagglutination titer after amplification no matter on chicken embryos or on cells. Thus in the present invention, the primary antigen proteins, i.e. HA and NA genes, of the ZJCIV canine influenza virus, and the other six internal genes of the PR8 virus are recombined together, so as to rescue, by means of the reverse genetic system of the influenza virus, a canine influenza recombinant virus that can generate very high virus titer and hemagglutination titer on both a chicken embryo and a cell, and this recombinant virus can be used as a good seed virus for developing canine influenza vaccines.

### EXAMPLE 1 Construction and Identification of the Recombinant Plasmids

### 1. PCR Amplification

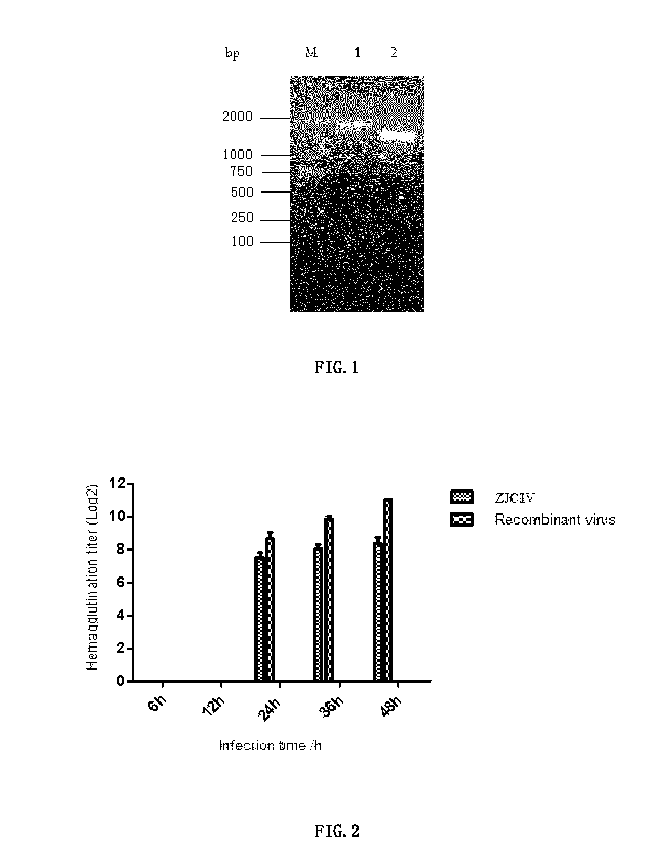

The total RNA of the canine influenza virus ZJCIV is extracted using Trizol (Invitrogen). With a 12-bp primer 5'-AGCAAAAGCAGG-3' serving as the specific primer, first-strand cDNA is synthesized using Reverse Transcription System Kit (TakaRa) according to its instruction. The HA and NA of the fragment ZJCIV are respectively amplified by taking the resultant first-strand cDNA as the template and taking sapl-HA-up, sapl-HA-down and sapl-NA-up, sapl-NA-down as upstream and downstream primers (containing *BspQI* enzyme digestion sites, as shown in Table 1). The PCR amplification procedure is as follows: pre-degenerate at 94°C for 5 minutes, enter the following cycle: degenerate at 94°C for 45 seconds, anneal at 53°C for 45 seconds and extend at 72°C for 1 minutes and 45 seconds, complete 30 cycles, and finally, extend at 72°C for 10 minutes. Meanwhile, a negative control with no template is established. At the end of reaction, PCR products undergo electrophoresis on 1.0% agarose gel. The results are shown in Fig.1: two PCR strips appear, i.e. HA having a size of about 1700bp and NA having a size of about 1400bp, and destination fragments are consistent in size. In Fig.1, M: DNA molecular weight marker; 1: ZJCIV HA PCR product; 2: ZJCIV NA PCR product.

**Table 1 Universal Primers of the HA and NA Genes of the Influenza A Virus**

| Primer Name | Primer Sequence |
|---|---|
| sapl-HA-up | CACACAgctcttctattAGCAAAAGCAGGGG (SEQ ID NO.5) |
| sapl-HA-down | CACACAgctcttcggccAGTAGAAACAAGGGTGTTTT (SEQ ID NO.6) |
| sapl-NA-up | CACACAgctcttctattAGCAAAAGCAGGAGT (SEQ ID NO.7) |
| sapl-NA-down | CACACAgctcttcggccAGTAGAAACAAGGAGTTTTTT (SEQ ID NO.8) |

| | |
|---|---|
| 2. Gel Cutting Recovery of the PCR Products | |

At the end of electrophoresis, the agarose gel of the destination DNA fragment is cut off from gel under ultraviolet light, and DNA is recovered by a DNA rapid recovery kit. The specific method is as follows: cut off the destination-DNA-containing agarose gel under a ultraviolet lamp, absorb all the liquid on the surface of the gel by tissue, cut the gel into pieces, put the gel pieces in a sterile 1.5ml EP tube, add Buffer DE-A (liquid gel) the volume of which is 3 times as much as that of the gel (100mg=100ul), mix uniformly and then heat at 75°C, mix intermittently (for 2-3 minutes) until the blocky gel is completely molten (about 6-8 minutes); add Buffer DE-B (binding buffer) the volume of which is a half of that of the Buffer DE-A, and mix uniformly. Add isopropanol the volume of which is equal to that of the gel when the recovered DNA fragment is less than 400bp. Transfer the mixed solution into a DNA preparation tube, centrifuge for 1 minute at a rate of 12000xg, and pour the waste solution in the collection tube. Place the preparation tube back into the collection tube, add 500ul Buffer W1 (cleaning solution), centrifuge for 30 seconds at a rate of 12000xg, and pour the waste solution in the collection tube. Place the preparation tube back into the collection tube, add 700ul Buffer W2 (desalting solution), centrifuge for 1 minute at a rate of 12000xg, pour the waste solution in the collection tube, and wash the preparation tube in the same way once again. Place the preparation tube back into the collection tube, and centrifuge in vacuum for 1 minute at a rate of 12000xg. Finally, place the preparation tube in the clean 1.5ml EP tube, add 30ul of de-ionized water to the center of a preparation membrane, stand for 1 minute under room temperature, centrifuge for 1 minute at a rate of 12000xg to elute DNA, and preserve the product at minus 20°Cfor future use.

### 3. Enzyme Digestion, Ligation and Transformation

According to the instruction, the purified PCR products and the PBD vectors (preserved in the lab) are incubated at 50°C for 1 hour under the action of *BspQ*I restriction endonuclease (NEB). The enzyme-digested products of the destination fragments and the PBD plasmids are recovered using a gel extraction kit, 1 ul of T4 ligase buffer solution and 1 ul of T4 ligase (TakaRa) are added, the reaction system for ligation is 10ul, and uniform mixing is carried out. Ligation is carried out overnight at 16°C The ligation product is transformed to a competent cell JM109 (prepared in the lab), and the competent cell is then coated on an Amp-containing LB solid culture medium in a clean bench under aseptic conditions, followed by culture for 8-20 hours at 37°C

### 4. Identification of the Recombinant Plasmids

A single colony on the LB solid culture medium is picked out and then placed in the test tube having Amp-containing LB liquid culture medium that has a volume of about 3ml, afterwards, the test tube is immobilized on a shaker for shaking culture at 37°C for 10 hours or overnight. The plasmids, which are extracted from the bacterial solution using an alkaline extraction method, are identified by a PCR method. The plasmids identified as positive undergo sequencing, and compared using DNAstar sequence analysis software. It turns out that the recombinant plasmids PBD-ZJCIVHA and PBD-ZJCIVNA are constructed successfully, the sequence of the HA gene is shown in SEQ ID NO.3 and the sequence of the NA gene is shown in SEQ ID NO.4.

### EXAMPLE 2 Rescue of the Recombinant PR8 Virus

### 1. Preparation for Plasmid Transfection

Plasmids are extracted using an ultra-pure plasmid extraction kit (OMEGA), and the operation steps are as follows: 1) dip preserved glycerin bacteria (containing plasmids PBD-ZJCIVHA, PBD-ZJCIVNA, PBD-PR8M, PBD-PR8PB1, PBD-PR8PB2, PBD-PR8PA, PBD-PR8NS and PBD-PR8NP, and the last six plasmids are preserved in the lab) using an inoculating loop, draw lines on the surface of an Amp-containing LB flat plate, and stand overnight at 37°C, 2) pick out and inoculate a single colony to a 5ml Amp LB culture medium, carry out shaking culture at 37°C until the OD600 value is 1.0-1.5; 3) collect 3ml of overnight culture and centrifuge the culture to remove the culture medium thoroughly; 4) bacteria suspension: carry out bacteria suspension using 0.25ml of RNase A-containing SolutionI until a homogenate state is reached; 5) cell lysis: add 0.25ml of SolutionII, reverse 5 times to achieve gentle and uniform mixing; 6) neutralization: add 0.125ml of Buffer N3, reverse 5 times immediately to achieve gentle and uniform mixing until white flocculent precipitates are formed, and centrifuge for 10 minutes at a rate of 12000xg under room temperature; 7) carefully pour the supernatant into a clean 1.5ml centrifuge tube, add to the supernatant an ETR Solution (blue) the volume of which is one-tenth of that of the centrifuge tube, reverse 7-10 times, and put in an ice bath for 10 minutes; 8) add a water bath at 42°C for 5 minutes, then mix to reach a turbid state, and centrifuge for 3 minutes at a rate of 12000xg under room temperature, so as to form a blue layer in the ETR solution at the bottom of the centrifuge tube; 9) transfer the supernatant into a new 1.5ml centrifuge tube, add absolute ethyl alcohol the volume of which is a half of that of the centrifuge tube, reverse 6-7 times, and stand for 1-2 minutes under room temperature; 10) pour the aforementioned mixed solution into a HiBand DNAMini column that is properly balanced by 2ml of E4 in advance, put the column on a 2ml collection tube, and centrifuge for 1 minute at a rate of 10000xg under room temperature so that lysis solution passes by the column; 11) discard the liquid in the collection tube, add the remaining mixed solution into the column, and centrifuge for 1 minute at a rate of 10000xg under room temperature so that lysis solution totally passes through the column; add 500ul of Buffer HB to the column, centrifuge for 1 minute at a rate of 10000xg under room temperature, and wash the column to ensure removal of the residual proteins, in order to obtain high-quality DNA; 12) discard the liquid, wash the column with 700ul of DNA Wash buffer, centrifuge for 1 minute at a rate of 10000xg under room temperature, and discard the liquid; 13) repeat the operation and then add DNA Wash buffer; 14) discard the liquid, carry out idling, centrifuge for 2 minutes at a rate of 12000xg under room temperature, and discard the liquid; 15) put the column in a clean 1.5ml centrifuge tube, add 30-50ul of endotoxin-free eluant onto the column, stand for 2 minutes under room temperature, and centrifuge for 1 minute at a rate of 12000xg under room temperature to elute DNA, wherein this elution can be carried out twice; and 16) carry out electrophoresis detection, and measure OD₂₆₀ and OD₂₈₀ using an Nanodrop 2000c ultraviolet-visible spectrophotometer to estimate DNA content and purity. Results: a sufficient amount of plasmids required by transfection are acquired.

### 2. Transfection of the 293T Cell

The aforementioned plasmids extracted at a ultra-purity, including PBD-ZJCIVHA, PBD-ZJCIVNA, PBD-PR8M, PBD-PR8PB1, PBD-PR8PB2, PBD-PR8PA, PBD-PR8NS and PBD-PR8NP, are co-transfected via a proper amount of liposome 2000 to a 293T cell having a diameter of 3.5cm. 6 hours after transfection, the cell supernatant is discarded, 2ml of OPTI-MEM (Invitrogen) culture solution is added, and the cell is put in a CO₂ incubator at 37°Cfor culture for 72 hours.

### 3. Rescue of the Recombined PR8 Virus

48 hours after transfection, the cell supernatant is inoculated to a 9-day to 11-day SPF chicken embryo (BEIJING MERIAL VITAL LABORATORY ANIMAL TECHNOLOGY CO., LTD.), the chicken embryo is sealed by paraffin and then put in an incubator at 37°C for culture. 48 to 72 hours later, the chicken embryo undergoes a temperature of 4°C overnight, and then taken out to obtain chicken embryo allantoic fluid. The presence of hemagglutinin activity in the allantoic fluid is determined by a hemagglutination test. Results: hemagglutination occurs in the allantoic fluid, indicating that a PR8 recombinant virus containing the HA and NA genes of the ZJCIV has been rescued successfully.

### 4. Identification of the Recombinant Virus

The total RNA of the allantoic fluid of the recombinant virus is extracted using Trizol, and then subjected to reverse transcription by a 12-bp primer to obtain first-strand cDNA. The HA and NA fragments of the ZJCIV are PCR-amplified by taking the first-strand cDNA as the template and taking sapl-HA-up, sapl-HA-down and sapl-NA-up, sapl-NA-down as upstream and downstream primers, and then identified by 1% agarose electrophoresis. In addition, the ZJCIV HA and ZJCIV NA PCR products undergo sequencing in the company. Results: on agarose electrophoresis gel, there are two strips having a size of about 1700bp and a size of about 1400bp respectively, which are completely consistent with the target sizes. And it is further proved by sequencing results that these PCR products are indeed the HA and NA fragments of the ZJCIV.

### EXAMPLE 3 Identification of the Growth Characteristics of the Rescued Recombinant Virus

### 1. Determination of EID₅₀ of the Rescued Recombinant Virus and the ZJCIV

The virus-containing chicken embryo allantoic fluid undergoes 10-fold dilutions, and the chicken embryo allantoic fluids that are diluted based on the dilutabilities from 10⁻⁶ to 10⁻¹⁰ are respectively inoculated to five 9-day to 11-day SPF chicken embryos for continuous incubation for 48 hours at 37°C. Whether the chicken embryo allantoic fluids are infected is judged by determining the hemagglutinin activities of the infected embryo allantoic fluids, and EID₅₀ (chicken embryos' median infective doses) is calculated using a Reed-Muench method. Results: EID₅₀ of the rescued recombinant virus and the ZJCIV are 10^{7.5}/100ul and 10^{6.5}/100ul, respectively.

### 2. Determination of TCID₅₀ of the Rescued Recombinant Virus and the ZJCIV

10-fold dilutions start from 1: 10⁻³, the recombinant virus and ZJCIV having different dilutabilities are inoculated to a 48-well plate on which monolayer MDCK cells grow, and the inoculation procedure is as follows: at first, clean the MDCK cells twice with PBS, then add 100ul of virus to each well, repeat this operation 3 times for every dilutability, put the 48-well plate in a CO₂ incubator at 37°C for the purpose that viruses are adsorbed on the cells, shake the cells left and right once at an interval of 20 minutes, discard the viruses in the cell culture plate 1.5-2.5 hours later, wash the cells twice with PBS, and then add 300ul of serum-free medium. Continuously culture the virus-adsorbed cells in the CO₂ incubator for 72 hours, then determine the hemagglutinin activity of every well, and calculate TCID₅₀ (tissue cells' median infective doses) using a Reed-Muench method. Results: TCID₅₀ of the rescued recombinant virus and the ZJCIV are 10^{6.5}/100ul and 10^{5.5}/100ul, respectively.

### 3. Growth Characteristic Comparison of the Rescued Recombinant Virus and the ZJCIV on the Chicken Embryos

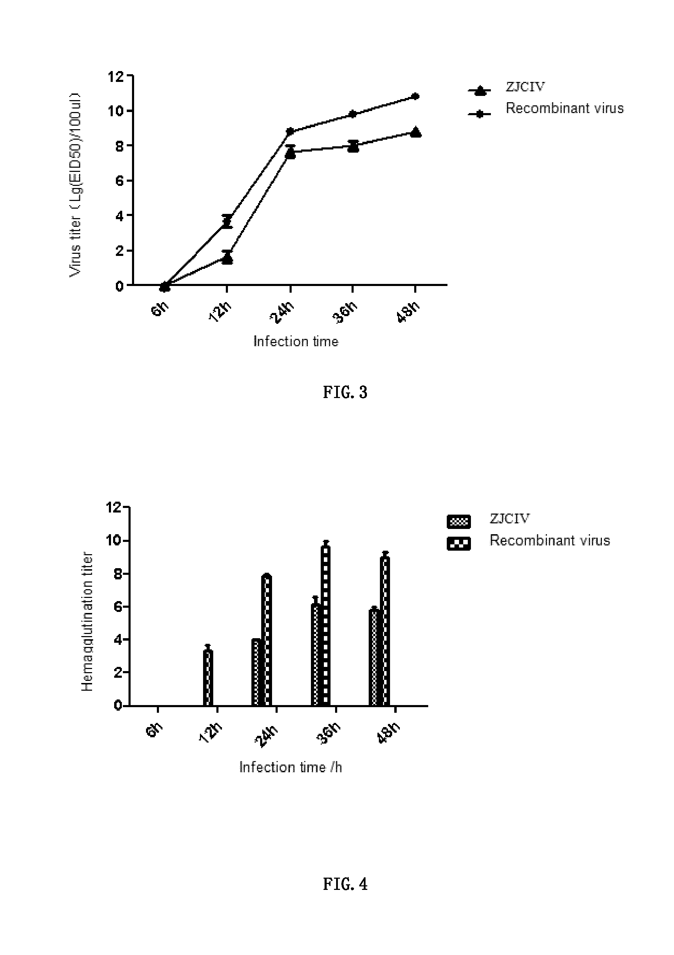

The rescued recombinant virus and the ZJCIV are diluted as 100EID₅₀, and are inoculated, based on this dilutability, to 18 9-day to 11-day SPF chicken embryos, respectively. 6 hours, 12 hours, 24 hours, 36 hours, 48 hours and 72 hours after inoculation, 3 inoculated SPF chicken embryos are taken out respectively, their allantoic fluids are collected and the hemagglutinin activities thereof are determined (Fig.2). The allantoic fluids collected at different time undergo 10-fold dilutions, the virus solution of each dilutability is inoculated to 3 9-day to 11-day SPF chicken embryos, and the inoculum dose is 100ul per chicken embryo. 48 hours after inoculation, the hemagglutinin activities of the chicken embryo allantoic fluids are determined, the virus contents of the allantoic fluids collected at different time are calculated, a growth curve of the virus is drawn up (Fig.3), and the growth conditions of the rescued recombinant virus and the ZJCIV on the chicken embryos are compared. The results are shown in Fig.2: the allantoic fluids of both the ZJCIV and the recombinant virus have no hemagglutination within 12 hours after inoculation. 48 hours after inoculation, the hemagglutination titer of the recombinant virus reaches 2¹¹, which is significantly higher than that of the ZJCIV. The results are shown in Fig.3: no virus is detected 6 hours after inoculation of the ZJCIV and the rescued recombinant virus. 12 hours after inoculation, the virus titer of the rescued recombinant virus is significantly higher than that of the ZJCIV, and reaches the peak 48 hours after inoculation.

### 4. Growth Characteristic Comparison of the Rescued Recombinant Virus and the ZJCIV on the MDCK cells

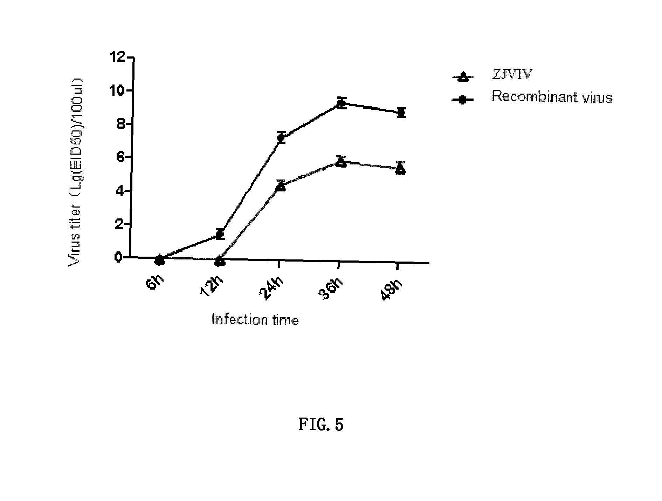

The rescued recombinant virus and the ZJCIV are diluted as 100TCID₅₀, and are each inoculated, based on this dilutability, to 3 T25 cell bottles in which 80% of the MDCK cells grow. 6 hours, 12 hours, 24 hours, 36 hours, 48 hours and 72 hours after inoculation, their cell supernatants are collected respectively and the hemagglutination titers thereof are determined (Fig.4). The virus contents in the cell supernatants collected at different time are titrated, specifically as follows: the collected cell supernatants undergo 10-fold dilutions, the virus solution of each dilutability is inoculated to the MDCK cells with 80% growing in 3 wells of a 24-well cell plate, the hemagglutinin activities are determined 48 hours after inoculation, the virus contents (TCID₅₀) in the cell supernatants collected at different time are calculated, a growth curve of the virus is drawn up according to this TCID₅₀ (Fig.5), and the growth conditions of the rescued recombinant virus and the ZJCIV on the MDCK cells are compared. The hemagglutination titer results are shown in Fig.4: within 12 hours after inoculation, the cell supernatant of the ZJCIV has no hemagglutinin activity, while the cell supernatant of the recombinant virus has hemagglutinin activity, which reaches the peak 36 hours after inoculation and subsequently tends to a relatively stable state. In the entire infection process, the hemagglutinin activity of the recombinant virus is significantly higher than that of the ZJCIV. The virus titer results are shown in Fig.5: no virus is detected 6 hours after inoculation of the ZJCIV and the rescued recombinant virus, and 12 hours after inoculation, the virus of the rescued recombinant virus can be detected, whereas the virus of the ZJCIV cannot be detected until 24 hours after inoculation. 36 hours after inoculation, the virus titers of the recombinant virus and the ZJCIV reach the peak and then begin descending. In the entire cell infection process, the virus titer of the recombinant virus is significantly higher than that of the ZJCIV.

The embodiments described above are for illustrating the ways of practicing the present invention only, and shall not be understood as limiting the patent scope of the present invention even if their descriptions are specific and detailed. It shall be noted that, many modifications and improvements could also be made by those ordinary skilled in this art without departing from the concept of the present invention, and these modifications and improvements shall fall within the scope of the present invention. Accordingly, the patent scope of the present invention shall be subject to the claims appended.

## Claims

1. A canine influenza recombinant virus comprising HA and NA genes of ZJCIV canine influenza virus as well as six internal genes PA, PB1, PB2, M, NP and NS of a PR8 virus,
the nucleotide sequence of the HA gene of the canine influenza virus is selected from the group consisting of:
(1) a nucleotide sequence encoding an amino acid sequence of SEQ ID NO.1;
(2) a nucleotide sequence encoding an amino acid sequence which has at least 98% sequence identity to the amino acid sequence of SEQ ID NO.1;
the nucleotide sequence of the NA gene of the canine influenza virus is selected from the group consisting of:
(1) a nucleotide sequence encoding an amino acid sequence of SEQ ID NO.2;
(2) a nucleotide sequence encoding an amino acid sequence which has at least 98% sequence identity to the amino acid sequence of SEQ ID NO.2.

2. The canine influenza recombinant virus according to claim 1, wherein the HA gene of the canine influenza virus has a nucleotide sequence of SEQ ID NO.3, or the HA gene of the canine influenza virus has a sequence having at least 98% sequence identity to the nucleotide sequence of SEQ ID NO.3.

3. The canine influenza recombinant virus according to claim 1, wherein the NA gene of the canine influenza virus has a nucleotide sequence of SEQ ID NO.4, or the NA gene of the canine influenza virus has a sequence having at least 98% sequence identity to the nucleotide sequence of SEQ ID NO.4.

4. A preparation method for the canine influenza recombinant virus, the method comprising:
constructing recombinant plasmids comprising the HA and NA genes of the ZJCIV canine influenza virus respectively;
transfecting the recombinant plasmids of the HA and NA genes, and six plasmids comprising the internal genes PA, PB1, PB2, M, NP and NS of the PR8 virus respectively, to a 293T cell, and culturing the transfected cell;
inoculating the cultured cell supernatant to a chicken embryo, culturing the chicken embryo in an incubator for a proper time period to obtain chicken embryo allantoic fluid, detecting the hemagglutination condition of the allantoic fluid, and at the presence of hemagglutinin activity, determining the absence of unexpected variations by sequencing to obtain the canine influenza recombinant virus.

5. The preparation method according to claim 4, wherein the recombinant plasmids contain PBD vectors used as empty vectors.

6. The preparation method according to claim 4, wherein the cultured cell supernatant is inoculated to a 9-day to 11-day chicken embryo, and the chicken embryo is cultured in a 37 °C incubator for 48-72 hours to obtain chicken embryo allantoic fluid.

7. Application of the canine influenza recombinant virus as claimed in claim 1, for preventing or treating canine influenza.

8. An influenza vaccine comprising the canine influenza recombinant virus as claimed in claim 1.


SEQUENCE LISTING

<110>  Shanghai Veterinary Research Institute, CAAS

<120>  Canine Influenza Recombinant Virus and Preparation Method Therefor
and Application Thereof

<160>  8

<170>  PatentIn version 3.3


<210>  1
<211>  566
<212>  PRT
<213>  canine influenza virus

<400>  1

Met Lys Thr Val Ile Ala Leu Ser Tyr Ile Phe Cys Leu Ala Phe Gly
1               5                   10                  15


Gln Asn Leu Pro Gly Asn Glu Asn Asn Ala Ala Thr Leu Cys Leu Gly
            20                  25                  30


His His Ala Val Pro Asn Gly Thr Ile Val Lys Thr Ile Thr Asp Asp
        35                  40                  45


Gln Ile Glu Val Thr Asn Ala Thr Glu Leu Val Gln Asn Ser Pro Thr
    50                  55                  60


Gly Lys Ile Cys Asn Asn Pro His Lys Ile Leu Asp Gly Arg Asp Cys
65                  70                  75                  80


Thr Leu Ile Asp Ala Leu Leu Gly Asp Pro His Cys Asp Val Phe Gln
                85                  90                  95


Asn Glu Thr Trp Asp Leu Phe Val Glu Arg Ser Asn Ala Phe Ser Asn
            100                 105                 110


Cys Tyr Pro Tyr Asp Val Pro Asp Tyr Ala Ser Leu Arg Ser Ile Val
        115                 120                 125


Ala Ser Ser Gly Thr Leu Glu Phe Ile Thr Glu Gly Phe Thr Trp Ala
    130                 135                 140


Gly Val Thr Gln Asn Gly Gly Ser Gly Ala Cys Lys Arg Gly Pro Ala
145                 150                 155                 160


Asn Gly Phe Phe Ser Arg Leu Asn Trp Leu Thr Lys Ser Gly Asn Thr
                165                 170                 175

Tyr Pro Val Leu Asn Val Thr Met Pro Asn Asn Asn Asn Phe Asp Lys
        180                 185                 190

Leu Tyr Ile Trp Gly Val His His Pro Ser Thr Asn Gln Glu Gln Thr
        195                 200                 205

Ser Leu Tyr Ile Gln Ala Ser Gly Arg Val Thr Val Ser Thr Arg Arg
    210                 215                 220

Ser Gln Gln Thr Ile Ile Pro Asn Ile Gly Ser Arg Pro Leu Val Arg
225                 230                 235                 240

Gly Gln Ser Gly Arg Ile Ser Val Tyr Trp Thr Ile Val Lys Pro Gly
                245                 250                 255

Asp Val Leu Val Ile Asn Ser Asn Gly Asn Leu Ile Ala Pro Arg Gly
            260                 265                 270

Tyr Phe Lys Met His Ile Gly Lys Ser Ser Ile Met Arg Ser Asp Ala
        275                 280                 285

Pro Ile Asp Thr Cys Ile Ser Glu Cys Ile Thr Pro Asn Gly Ser Ile
    290                 295                 300

Pro Asn Glu Lys Pro Phe Gln Asn Val Asn Lys Ile Thr Tyr Gly Ala
305                 310                 315                 320

Cys Pro Lys Tyr Val Lys Gln Asn Thr Leu Lys Leu Ala Thr Gly Met
                325                 330                 335

Arg Asn Val Pro Glu Arg Gln Thr Arg Gly Leu Phe Gly Ala Ile Ala
            340                 345                 350

Gly Phe Ile Glu Asn Gly Trp Glu Gly Met Val Asp Gly Trp Tyr Gly
        355                 360                 365

Phe Arg His Gln Asn Ser Glu Gly Thr Gly Gln Ala Ala Asp Leu Lys
    370                 375                 380

Ser Thr Gln Ala Ala Ile Asp Gln Ile Asn Gly Lys Leu Asn Arg Met
385                 390                 395                 400

Ile Glu Lys Thr Asn Glu Lys Phe His Gln Ile Glu Lys Glu Phe Ser
                405                 410                 415

Glu Val Glu Gly Arg Ile Gln Asp Leu Glu Arg Tyr Val Glu Asp Thr
            420                 425                 430

Lys Val Asp Leu Trp Ser Tyr Asn Ala Glu Leu Leu Val Ala Leu Glu
        435                 440                 445

Asn Gln Asn Thr Ile Asp Leu Thr Asp Ser Glu Met Asn Lys Leu Phe
    450                 455                 460

Glu Lys Thr Arg Arg Gln Leu Arg Glu Asn Ala Glu Asp Met Gly Asn
465                 470                 475                 480

Gly Cys Phe Lys Ile Tyr His Lys Cys Asp Asn Ala Cys Ile Glu Ser
                485                 490                 495

Ile Arg Asn Gly Thr Tyr Asp His Asn Ile Tyr Arg Asp Glu Ala Val
            500                 505                 510

Asn Asn Arg Phe Gln Ile Lys Gly Val Glu Leu Lys Ser Gly Tyr Lys
        515                 520                 525

Asp Trp Ile Leu Trp Ile Ser Phe Ala Ile Ser Cys Phe Leu Leu Cys
    530                 535                 540

Val Val Leu Leu Gly Phe Ile Met Trp Ala Cys Gln Arg Gly Asn Ile
545                 550                 555                 560

Arg Cys Asn Ile Cys Ile
                565

<210> 2
<211> 471
<212> PRT
<213> Canine influenza virus

<400> 2

Met Asn Pro Asn Gln Lys Ile Ile Ala Ile Gly Ser Val Ser Leu Thr
1               5                   10                  15

Ile Ala Thr Val Cys Phe Leu Leu Gln Ile Ala Ile Leu Ala Thr Thr
            20                  25                  30

Val Thr Leu Tyr Phe Lys Gln Asn Glu Cys Asn Ile Pro Ser Asn Ser
        35                  40                  45

Gln Val Val Pro Cys Lys Pro Ile Ile Ile Glu Arg Asn Ile Thr Glu
    50                  55                  60

Val Val Tyr Leu Asn Asn Thr Thr Ile Glu Lys Glu Lys Glu Ile Cys
65                  70                  75                  80

Ser Val Val Leu Glu Tyr Arg Asn Trp Ser Lys Pro Gln Cys Gln Ile
                85                  90                  95

Thr Gly Phe Ala Pro Phe Ser Lys Asp Asn Ser Ile Arg Leu Ser Ala
            100                 105                 110

Gly Gly Asp Ile Trp Val Thr Arg Glu Pro Tyr Val Ser Cys Asp Pro
        115                 120                 125

Ser Lys Cys Tyr Gln Phe Ala Leu Gly Gln Gly Thr Thr Leu Asn Asn
    130                 135                 140

Lys His Ser Asn Gly Thr Ile His Asp Arg Ile Ser His Arg Thr Leu
145                 150                 155                 160

Leu Met Asn Glu Leu Gly Val Pro Phe His Leu Gly Thr Lys Gln Val
                165                 170                 175

Cys Ile Ala Trp Ser Ser Ser Ser Cys His Asp Gly Lys Ala Trp Leu
            180                 185                 190

His Val Cys Val Thr Gly Asp Asp Arg Asn Ala Thr Ala Ser Phe Val
        195                 200                 205

Tyr Asn Gly Met Leu Val Asp Ser Ile Gly Ser Trp Ser Gln Asn Ile
    210                 215                 220

Leu Arg Thr Gln Glu Ser Glu Cys Val Cys Ile Asn Gly Thr Cys Thr
225                 230                 235                 240

Val Val Met Thr Asp Gly Ser Ala Ser Gly Arg Ala Asp Thr Arg Ile
                245                 250                 255

Leu Leu Ile Arg Glu Gly Lys Ile Val His Ile Ser Pro Leu Ser Gly
            260                 265                 270

Ser Ala Gln His Ile Glu Glu Cys Ser Cys Tyr Pro Arg Tyr Pro Asn
        275                 280                 285

Val Arg Cys Val Cys Arg Asp Asn Trp Lys Gly Ser Asn Arg Pro Val
    290                 295                 300

Ile Asp Ile Asn Met Ala Asp Tyr Ser Ile Asp Ser Ser Tyr Val Cys
305                 310                 315                 320

Ser Gly Leu Val Gly Asp Thr Pro Arg Asn Asp Asp Ser Ser Ser Ser
                325                 330                 335

Ser Asn Cys Arg Asp Pro Asn Asn Glu Arg Gly Asn Pro Gly Val Lys
            340                 345                 350

Gly Trp Ala Phe Asp Asn Glu Asn Asp Val Trp Met Gly Arg Thr Ile
        355                 360                 365

Ser Arg Asn Leu Arg Ser Gly Tyr Glu Thr Phe Lys Val Ile Gly Gly
    370                 375                 380

Trp Thr Thr Ala Asn Ser Lys Ser Gln Val Asn Arg Gln Val Ile Val
385                 390                 395                 400

Asp Asn Asn Asn Trp Ser Gly Tyr Ser Gly Ile Phe Ser Val Glu Gly
                405                 410                 415

Lys Ser Cys Val Asn Arg Cys Phe Tyr Val Glu Leu Ile Arg Gly Gly
            420                 425                 430

Pro Gln Glu Thr Arg Val Trp Trp Thr Ser Asn Ser Ile Val Val Phe
        435                 440                 445

Cys Gly Thr Ser Gly Thr Tyr Gly Thr Gly Ser Trp Pro Asp Gly Ala
    450                 455                 460

Asn Ile Asn Phe Met Pro Ile
465                 470

<210>  3
<211>  1701
<212>  DNA
<213>  canine influenza virus

<400>  3
atgaaaactg ttattgcttt aagctacatt ttctgcctgg cttttggtca gaatcttcca      60

ggaaatgaaa ataatgctgc aacactatgc ctgggacatc atgcagtgcc gaacggaaca     120

atagtgaaaa ctatcacaga cgatcaaatt gaggtgacca acgccaccga gctagtccaa     180

aactccccaa cagggaaaat atgcaacaat ccccacaaga ttcttgatgg gagggactgc     240

acactaatag atgccctact aggggacccg cactgtgacg tcttccaaaa tgagacatgg     300

gacctttttg tggaacgaag caatgctttt agcaattgtt acccttatga tgtaccagac     360

tatgcatccc ttcgatccat agttgcatca tcaggcacat tggagttcat cactgaaggt     420

ttcacttggg caggagtaac tcaaaatgga ggaagcggtg cttgtaaaag gggacctgct     480

aatggtttct tcagtagatt gaattggtta actaagtcag gaaatacata tccagtgttg     540

aatgtgacta tgccaaacaa taacaatttc gacaaattat acatttgggg agttcatcac     600

ccaagcacta atcaagaaca aaccagcctg tatattcagg cctcaggaag agtcacagtc 660

tctaccagga gaagccaaca gaccataatc ccaaacattg gatctagacc cttggtaagg 720

ggccaatctg gcagaataag cgtatattgg acaatagtca aacctggaga cgtactggta 780

ataaacagta atggaaacct aatcgctcct cgaggctact tcaaaatgca cattgggaaa 840

agctcaataa tgagatcaga tgcgcctatt gacacctgca tttccgaatg tatcactccg 900

aacgggagca tccccaatga aaagcccttc caaaatgtga acaagatcac atacggagca 960

tgtcccaaat atgttaagca aaacaccttg aaactggcaa caggaatgcg gaatgtccct 1020

gagaggcaaa ccagaggcct gttcggcgca atagcaggct tcatagaaaa tggatgggaa 1080

gggatggtag acggttggta tggcttcagg caccaaaatt ccgaaggtac aggacaagca 1140

gcagacctta aaagcactca ggcagccatt gaccagatta atgggaaatt gaacagaatg 1200

attgaaaaaa cgaatgagaa gttccatcaa atcgaaaagg agttttccga agtagaaggg 1260

aggattcaag accttgagag atacgttgaa gacacaaaag tagatctttg gtcttacaat 1320

gccgagcttc ttgttgcttt agaaaaccag aacacaattg atttaactga ttcagaaatg 1380

aacaaattgt ttgaaaagac taggaggcaa ttgagggaaa atgctgaaga catgggcaat 1440

ggctgcttca agatatacca caagtgtgac aatgcttgca tagaatcgat tagaaacgga 1500

acttatgacc ataacatata tagagatgag gcagtgaaca atcggttcca gatcaaaggt 1560

gttgagctaa agtctggata caaagactgg atcttgtgga tttcctttgc catatcatgc 1620

tttttgcttt gtgttgtctt gctgggtttc attatgtggg cctgccagag aggcaacatt 1680

aggtgcaaca tttgcatttg a 1701

<210> 4
<211> 1416
<212> DNA
<213> canine influenza virus

<400> 4
atgaacccaa atcaaaagat aatagcaata ggttctgtct ctctaaccat tgcaacagta 60

tgtttcctct tgcagattgc catcctagca acaactgtga cactgtactt caagcaaaat 120

gaatgcaaca tcccctcgaa cagtcaagta gtgccatgta aaccaatcat aatagaaagg 180

aacataacag aggtagtata tttgaataat actaccatag aaaaagaaaa agaaatttgt 240

tccgtagtgc tagaatacag gaactggtcg aaaccgcagt gtcaaattac aggatttgct 300

cctttctcca aggacaactc aatccgactc tccgctggtg gggacatttg ggtaacaagg 360

gaaccttatg tgtcatgcga ccccagcaaa tgttatcagt ttgcacttgg gcaggggacc 420

acgctgaaca ataaacactc aaacggcaca atacatgata ggatctctca tcgaactctt 480

ttaatgaatg agttgggtgt tccgtttcat ttgggaacca aacaagtgtg catagcatgg 540

tccagttcaa gttgtcacga tgggaaagca tggttacatg tttgtgtcac tggggatgat 600

agaaatgcga ctgctagttt cgtttataat ggaatgcttg ttgacagtat tggttcatgg 660

tctcaaaata tcctcagaac tcaagagtca gaatgcgttt gcatcaatgg aacttgtaca 720

gtagtaatga ctgatggaag tgcatcagga agggctgata ctagaatact actcatcaga 780

gaggggaaaa ttgtccatat tagcccattg tcagggagtg ctcaacatat agaggaatgt 840

tcctgttatc ctcgatatcc aaatgttaga tgtgtttgca gagacaattg gaagggctct 900

aataggcccg ttatagatat aaatatggca gattatagca tcgattccag ttatgtgtgt 960

tcaggacttg ttggcgatac accaaggaat gatgatagct ctagcagcag taactgcagg 1020

gatcctaata atgagagagg gaatccagga gtgaaagggt gggcctttga taatgagaat 1080

gacgtttgga tggggaggac aatcagcaga aatttgcgct caggttatga gactttcaag 1140

gtcattggtg gctggaccac tgctaattcc aagtcacagg tcaatagaca agtcatagtt 1200

gacaataata actggtctgg ttattctggt attttctccg ttgaaggcaa aagctgtgtt 1260

aataggtgtt tttatgtaga gttgataaga ggagggccac aagagactag agtatggtgg 1320

acttcaaata gcattgtcgt attttgtggt acttctggta cctatggaac aggctcatgg 1380

cctgatgggg cgaatatcaa cttcatgcct atataa 1416

<210> 5
<211> 31
<212> DNA
<213> Artificial Sequence

<220>
<221> misc_feature
<222> (1)..(31)
<223> primer

<400> 5
cacacagctc ttctattagc aaaagcaggg g 31

<210> 6
<211> 37
<212> DNA
<213> Artificial Sequence

<220>
<221> misc_feature
<222> (1)..(37)
<223> primer

<400> 6
cacacagctc ttcggccagt agaaacaagg gtgtttt 37

<210> 7
<211> 32

<212>  DNA
<213>  Artificial Sequence

<220>
<221>  misc_feature
<222>  (1)..(32)
<223>  primer

<400>  7
cacacagctc ttctattagc aaaagcagga gt                                    32

<210>  8
<211>  38
<212>  DNA
<213>  Artificial Sequence

<220>
<221>  misc_feature
<222>  (1)..(38)
<223>  primer

<400>  8
cacacagctc ttcggccagt agaaacaagg agtttttt                              38